(19) Europäisches Patentamt
European Patent Office
Office européen des brevets

(11) Publication number: **0 392 648 A1**

# (12) EUROPEAN PATENT APPLICATION

(21) Application number: **90300430.7**

(22) Date of filing: **16.01.90**

(51) Int. Cl.[5]: **C07C 327/38, A01N 55/02, A01N 37/28**

The title of the invention has been amended (Guidelines for Examination in the EPO, A-III, 7.3).

(30) Priority: **30.01.89 GB 8901996**

(43) Date of publication of application: **17.10.90 Bulletin 90/42**

(84) Designated Contracting States: **AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Austin, Peter William**
**45 Randale Drive**
**Bury BL9 8NF, Lancashire(GB)**

(74) Representative: **James, David Gomer et al**
**Imperial Chemical Industries PLC Legal Department: Patents Po Box 6 Bessemer Road**
**Welwyn Garden City Herts, AL7 1HD(GB)**

## (54) Metal complexes, the preparation thereof and use as biocides.

(57) A metal complex of the general formula

$[R^1CSNRO]_xM(L)_y$

where R and $R^1$ are optionally substituted hydrocarbyl groups, M is a metal of Group IIIA to VA and IB to VIIB, with the exception of copper, L is a ligand, x has a value of 1 to 3 and y is zero or has a value of up to 4. The complex may be prepared by the reaction of a metal salt with an N-hydroxythiobenzamide. Complexes of this type have anti-microbial properties, and can be used as industrial biocides.

EP 0 392 648 A1

## COMPOUND AND USE

The present invention relates to a class of compounds, the preparation of such compounds, and the use of the compounds as industrial biocides.

Industrial biocides are useful to prevent industrial spoilage, in particular that caused by bacteria and fungi. Materials which can be used as industrial biocides have antimicrobial properties and particularly have antifungal or antibacterial properties or preferably both antifungal and antibacterial properties. Such materials are useful in the preservation of paints, lactices, adhesives, leather, wood, metal working fluids and cooling water.

British Patent Specification No.1113634 discloses fungicidal compositions comprising an isothiazolothione in admixture with a solid diluent or a liquid diluent containing a surface active agent. The isothiazolothione is of the formula:

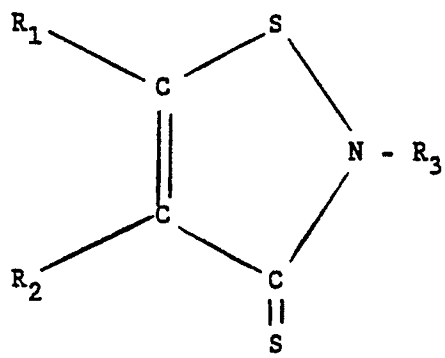

wherein $R_1$ and $R_2$ may, inter alia, together with their adjacent carbon atoms, constitute a ring. such ring systems include a cyclopentene or cyclohexene ring (compounds 8, 9 and 10) or a benzene ring (compounds 11 to 43). However, it is indicated that such compounds may isomerise to a structure containing an oxime group. The structure of compounds of this type, such as compound 41 as disclosed in GB 1113634, has been studied and it is concluded, in Il. Farmaco-Ed.Sci., Vol.23, pages 572 to 582, that in such compounds the oxime structure is more stable.

British Patent Specification No. 1104893 discloses a biocidal composition in which the active ingredient is disclosed as being at least one 3-imino-1,2-dithiole derivatives, such as, for example 3H-1,2-benzodithiol-3-one oxime and 4,5,6,7-tetrahydro-3H-1,2-benzo-dithiol-3-one oxime.

Cyclic compounds containing a thione group have been described in the literature but few of these other references indicate that the compounds possessed any antimicrobial activity.

US Patent 3448116 discloses, as anticonvulsants, compounds such as 1-hydroxyhydantoins and 1-hydroxythiohydantoins. J.C.S.Perkin 2, (1981), page 92ff; Chem.Ber. (1964), 97, page 216ff; Chem.Ber. (1971), 104, page 1512ff; and Arch.Pharm. (1978), 311(1), page 39ff describe cyclic compounds containing a thione group and having two nitrogen atoms in the ring adjacent to the thione group. J.C.S.Perkin 1 (1986) pages 39 to 59 discloses, inter alia, N-hydroxythiazolinthione derivatives and the preparation thereof. In our European Patent Application Publication No. 0249328 we disclose certain cyclic thiohydroxamic acid derivatives and the metal complexes thereof and the use of such compounds as anti-microbial agents. These compounds include 3-hydroxy-4-methyl-thiazol-2(3H)-thione and derivatives thereof including metal complexes and salts such as the zinc complex of 3-hydroxy-4-methyl-thiazol-2(3H)-thione.

Metal complexes of acyclic thiohydroxamic acids are disclosed in Aceta.Chem.Scand.1967 (21), page 1936 and Australian Journal of chemistry 1977 (30) page 2439. In J.Antibiot 1970 (23) 546 and 1971 (24) 124, the iron and copper complexes of N-methyl-N-hydroxythioformamide are disclosed as naturally occurring antibiotics.

We have now found a class of compounds which have useful anti-microbial properties.

According to the present invention there is provided a complex of the general formula

$[R^1CSNRO]_xM(L)_y$

where

R and $R^1$, which may be the same or different, are hydrocarbyl groups, or substituted hydrocarbyl groups;

M is a metal of Group IIIA to VA or IB to VIIB, with the exception of copper;

L represents a ligand;

x has a value of from 1 to 3; and
y is zero or has a value of from 1 to 4.

The groups R and $R^1$ may be alkyl, cycloalkyl, aryl, aralkyl, or alkaryl groups and typically contain from 1 up to 10 carbon atoms. If one, or both, of the groups R and $R^1$ is substituted, the, or each, substituent may be a hydrocarbonoxy group; an acyl group; an ester (that is an acryloxy) group, a halogen atom or a nitrile group. Alternatively, the substituent may be a heteroatom in a heterocyclic group, which may itself be substituted with substituents as herein before described. Heterocyclic groups include pyridyl, thienyl, imidazoly, and thiazoyl. If R and/or $R^1$ is a cyclic, including heterocyclic, group, this typically contains at least five, and particularly at least six, atoms. The groups R and $R^1$ may be the same but typically are different. Typically R is a lower alkyl group such as a methyl group and it is preferred that R is not an alkaryl group, especially one in which there is only one carbon atom between the aryl group and the nitrogen atoms. $R^1$ can be an alkyl group, especially a lower alkyl group such as methyl, ethyl, n-propyl or i-propyl or can be a cyclic group such as a phenyl group.

The metal M is a metal of Groups IIIA to VA or IB to VIIB of the Periodic Table, with the exception of copper. All references herein to the Periodic Table are to the Periodic Table according to Mendeleefff, as set out on the inside rear cover of "Handbook of Chemistry and Physics" 49th Edition (1968-1969) published by The Chemical Rubber Co. Cleveland, Ohio, USA. We have obtained compounds having useful properties when the metal is a metal of Group IIB, for example zinc. Useful results have also been obtained when the metal is a metal of Group IB, other than copper, for example silver.

The ligand L is typically a neutral or anionic ligand. Suitable ligands include halide, for example, chloride, water, alcohols, ketones, carboxylic acids, amines, sulphoxides and the like. The ligand, if present, typically results from the reagents or solvent used to prepare the metal complex and, in particular, the ligand results from the solvent used during the preparation of the metal complex. The ligand may be a mixture of anionic groups and neutral ligands.

The value of x typically corresponds to the valency of the metal M but does not exceed three. When the metal M is a metal of Group IIB, the value of x is two.

The value of y may be zero or may have a value of up to four. If the ligand group is an anionic group, the value of y should be such that the sum (x + y) corresponds to the valency of the metal M. The value of y should be such that the substituent groups in the complex are sufficient in number to satisfy the co-ordination number of the metal M. If the metal M is a metal of Group IIB, the co-ordination number of such metals is generally four and hence in such complexes the value of x is two and the value of y is zero.

One useful complex in accordance with the present invention is the 2:1 complex obtained from N-methyl-N-hydroxythiobenzamide and zinc, that is a complex in which R is methyl, $R^1$ is phenyl, M is zinc, x is two and y is zero. Other useful complexes are 2:1 complexes obtained from N-methyl-N-hydroxythioacetamide, N-methyl-N-hydroxythiopropionamide, N-methyl-N-hydroxythioisobutyramide or N-methyl-N-hydroxythiobutyramide and zinc, that is complexes in which R is methyl, M is zinc, x is two, y is zero and $R^1$ is methyl, ethyl, isopropyl or n-propyl. The 1:1 complex obtained from N-methyl-N-hydroxythiobenzamide and silver has also been found to have useful properties.

The complex of the present invention can be prepared using known techniques for the preparation of metal complexes. Conveniently the metal complex is prepared by the reaction of a salt of the metal with an N-hydroxythioamide. More specifically, a salt of the metal M is reacted with an N-hydroxythioamide of the formula

$R^1CSNROH$

where M, R and $R^1$ are as hereinbefore defined.

The salt of the metal M is preferably used as a solution in a suitable solvent. Thus, the salt may be an acetate such as zinc acetate which is dissolved in an alcohol, for example methanol. Other salts may be used, for example silver nitrate in the preparation of the silver complex.

The N-hydroxythioamide may be N-methyl-N-hydroxythio-benzamide, N-methyl-N-hydroxythioacetamide, N-methyl-N-hydroxythiopropionamide, N-methyl-N-hydroxythioisobutyramide and N-methyl-N-hydroxythiobutyramide. A less preferred N-hydroxythioamide is N-benzyl-N-hydroxythioacetamide. N-hydroxythioamides may be prepared using known procedures, for example as described in Acta Chemica Scand 1967 (21) 1936.

The reaction is conveniently effected by mixing together solutions, in the same solvent, of the salt of the metal and the N-hydroxythioamide. The solutions can be mixed together without heating the mixture. However, whilst the reaction can be effected at essentially ambient temperature (15-20°C), higher or lower temperatures may be used, for example from 0°C up to 100°C, although it is generally not preferred to use a temperature in excess of 50°C.

The reaction is preferably effected in a liquid which is a solvent for the reactants but a non-solvent for

the metal complex obtained. The metal complex is typically a solid and is formed as a precipitate during the reaction. The solid is readily separated from the reaction mixture, for example by filtration. The solid is then washed to remove impurities, for example using water, the solvent used for the preparation or both in sequence and/or as a mixture.

The reaction is conveniently effected by mixing together solutions of the two reactants and stirring to effect reaction. If the metal complex separates as a solid, stirring of the reaction mixture is continued for 0.1 up to 10 hours, for example 0.5 up to 2 hours. Stirring is then terminated and the solid is separated, conveniently by filtration but other techniques such as allowing the solid to settle and removing the supernatant liquid phase may also be used.

The metal salt and the N-hydroxythioamide are conveniently reacted together in essentially the stoichiometric quantities required to obtain the desired metal complex. Thus, the molar ratio of the N-hydroxythioamide to the metal salt is typically 0.9x:1 to 1.1x:1 and especially is 0.95x:1 to 1.05x:1, where x is as defined herein.

The metal complexes of the present invention have antimicrobial properties and, in particular, show considerable activity against fungi. Metal complexes of the present invention show increased antimicrobial activity compared to that of the corresponding copper complex. The metal complexes of the present invention may be used alone as an antimicrobial material but may also be used in, or on, a suitable carrier material.

Thus, as a further aspect of the present invention there is provided a biocide composition comprising a carrier and an effective amount of at least one metal complex of the formula:

$[R^1CSNRO]_xM(L)_y$

wherein R, $R^1$, M x and y are all as hereinbefore defined.

The carrier is typically a material which shows little, if any, antimicrobial activity and may be, or include, a material which is susceptible to the growth of micro-organisms, particularly fungi and bacteria. The carrier is preferably a liquid medium and the biocide composition may be a solution, suspension or emulsion of the metal complex in a liquid carrier. The carrier may be water, in which the metal complex is essentially insoluble, or may be a liquid such as acetic acid, N,N-dimethylformamide, propylene glycol, dimethyl sulphoxide or N-methyl-2-pyrrolidone in which the metal complex is soluble. Alternatively, a mixture of liquids may be used, one being a solvent for the metal complex and the other being a non-solvent, and using such a mixture the composition typically comprises an emulsion or droplets of a solution of the metal complex in the solvent therefor dispersed in the non-solvent. If a suspension or emulsion is used, this conveniently contains a surface active agent which is effective to maintain the non-continuous phase as a suspension or emulsion. Any surface active agent which is effective as a dispersant or emulsifier and is known for use in biocide compositions may be used such as, for example, alkylene oxide copolymers, and alkylene oxide adducts of fatty alcohols, alkyl phenols and amines such as ethylene diamine. Specific surface active agents which can be used include sodium lignosulphonate, EO/PO/EO block copolymers, ethylene oxide condensates with nonyl phenol or beta-naphthol, PO/EO copolymer condensates with nonyl phenol or ethylene diamine and condensates of naphthalene beta-sulphonic acid and formaldehyde. The surfactant is typically present in an amount of from 0.1 to 20% by weight of the weight of the total dispersion or emulsion in which the surfactant is to be incorporated. The dispersion or emulsion may include, in addition to the surfactant, other components which are known for inclusion in biocide compositions such as thickening agents. Materials which can be used as thickening agents include polysaccharide xanthan gum, sodium magnesium silicate, heteropolysaccharide, alginates, carboxymethyl cellulose, gum arabic, polyacrylic acid and polyvinyl alcohol.

Alternatively, or additionally, the biocide composition may include one or more solid components which may act as carriers or diluents. Solid materials which may be used as the optional component include inorganic materials such as metal oxides or mixtures or compounds thereof, for example aluminium oxide, silicon oxide, titanium dioxide, zinc oxide, talc, pyrophyllite, gypsum, kieselguhr, chalk, diatomaceous earth, bentonite and ufller's earth and organic materials such as wheat flour, soybean flour, wood flour, walnut shell flour and lignin. The solid material is preferably in a finely divided form and typically has an average particle size of not more than 5 micrometres. Any optional solid may be included in the biocide composition in an amount of from 1% up to 95% by weight of the total weight of the biocide composition, including the optional solid, and in general the optional solid will be present in an amount of at least 10% and not more than 80% by weight of the biocide composition.

The biocide composition may include a de-dusting agent, particularly if the composition is in a solid form. Suitable de-dusting agents include dodecyl benzene, tridecyl octadecanoate, trimethylol propane tridodecenoate, twitchel oil, Ensitol USN and mineral oil.

The amount of the metal complex which is present in the biocide composition may be just sufficient to

have an antimicrobial effect or the metal complex may be present in a substantially greater proportion. It will be appreciated that the biocide composition may be provided as a concentrated solution, suspension or emulsion which is subsequently diluted for use as an antimicrobial material. Thus, the amount of the metal complex which is present in the biocide composition is typically in the range from 0.0001% up to 30% and especially up to 10%, by weight of the biocide composition.

The biocide composition has antimicrobial activity and is especially effective in providing anti-bacterial or anti-fungal activity or both. Thus, the compositions can be used for the treatment of various media to inhibit the growth of micro-organisms.

As a further aspect of the present invention there is provided a method for inhibiting the growth of micro-organisms on, or in, a medium which comprises treating the medium with a metal complex as hereinbefore defined. The medium may be treated with a biocide composition which contains the metal complex.

The metal complex can be used in conditions in which micro-organisisms, especially bacteria or fungi, grow and cause problems. Systems in which micro-organisms cause problems include liquid, particularly aqueous, systems such as cooling water liquors, metal working fluids, geological drilling lubricants, polymer emulsions and surface coating compositions such as paints, varnishes and lacquers and also solid materials such as wood and leather..The metal complex can be included in such materials and is particularly useful when incorporated into a paint, varnish or lacquer to which they can provide protection against bacteria during storage and/or anti-fungal characteristics.

As a particular aspect of the present invention there is provide a surface coating composition which contains an effective amount of a metal complex in accordance with the present invention.

The surface coating composition may be a paint, varnish or lacquer and is especially a paint, for example an emulsion paint. The amount of the metal complex which is present in the surface coating composition is typically in the range from 0.001 up to 2% by weight and especially 0.1 up to 1% by weight relative to the total weight of the surface coating composition excluding the metal complex. The metal complex provides protection against bacteria during storage and can provide anti-fungal properties when the surface coating composition has been applied to a surface.

We have also found the metal complexes in accordance with the present invention give a useful effect when incorporated into a metal working fluid.

Thus, as a further aspect, the present invention provides a metal working fluid which contains an effective amount of a metal complex in accordance with the present invention. The amount of the metal complex which is present in the metal working fluid is typically in the range from 0.05 up to 2% by weight and especially 0.1 up to 1% by weight relative to the total weight of the metal working fluid, excluding the metal complex. The metal complex provides improved resistance to bacterial growth.

The metal complex of the present invention may be the only antimicrobial compound or may be used together with other compounds having antimicrobial characteristics. Thus, a mixture of different metal complexes in accordance with the present invention may be used. Alternatively, at least one metal complex in accordance with the present invention may be used together with one or more known antimicrobial compounds. The use of a mixture of anti-microbial compounds can provide a composition having a broader anti-microbial spectrum and hence one which is more generally effective than the components thereof. The known antimicrobial may be one possessing anti-bacterial, anti-fungal, anti-algal or other antimicrobial characteristic. The mixture of the metal complex of the present invention with other antimicrobial compounds typically contains from 1 to 99% by weight, relative to the weight of total antimicrobially active compounds, of the metal complex, particularly from 40 to 60% by weight of the metal complex.

As examples of known antimicrobial compounds which may be used, together with the metal complex of the present invention, there may be mentioned quaternary ammonium compounds such as diethyldodecylbenzyl ammonium chloride; dimethyloctadecyl-(dimethylbenzyl)ammonium chloride; dimethyldidecylammonium chloride; dimethyldidodecylammonium chloride; trimethy-tetradecylammonium chloride; benzldimethyl($C_{12}$-$C_{18}$ alkyl)ammonium chloride; dichlorobenzyldimethyldodecylammonium chloride; hexadecylpyridinium chloride; hexadecylpyridinium bromide; hexadecyltrimethylammonium bromide; dodecylpyridinium chloride; dodecylpyridinium bisulphate; benzyldodecyl-bis(beta-hydroxyethyl)ammonium chloride; dodecylbenzyltrimethylammonium chloride; benzyldimethyl($C_{12}$-$C_{18}$ alkyl) ammonium chloride; dodecyldimethylethyl ammonium ethylsulphate; dodecyldimethyl-(1-naphthylemthyl)ammonium chloride; hexadecyldimethylbenzyl ammonium chloride; dodecyldimethylbenzyl ammonium chloride and 1-(3-chloroallyl)-3,5,7-triaza-1-azonia-adamantane chloride; urea derivatives such as 1,3-bis(hydroxymethyl)-5,5-dimethylhydantoin; bis(hydroxymethyl)urea; tetrakis(hydroxymethyl)acetylene diurea; 1-(hydroxymetnhyl)-5,5-dimethylhydantoin and imidazolidinyl urea; amino compounds such as 1,3-bis(2-ethylhexyl)-5-methyl-5-aminohexahydropyrimidine; hexamethylene tetra amine; 1,3-bis(4-aminophenoxy)propane; and 2-[-(hydroxymethyl)-amino]ethanol; imidazole derivatives such as 1[2-(2,4-dichlorophenyl)-2-(2-propenyloxy)-ethyl]1H-imidazole; 2-(methoxycarbonylamino)-benzimidazole; nitrile compounds such as 2,4,5,6-tetrachloroisophthalodinitrile and 1,2-dibromo-2,4-dicyanobutane; thiocyanate derivatives such as methylene bis thiocyanate; zinc compounds or complexes such as zinc-2-pyridinethiol-N-oxide; tin compounds or complexes such as tributyltin-oxide, chloride, naphthoate, benzoate or 2-hydroxybenzoate; thiazole derivatives such as 2-(thiocyanomethylthio)-benzthiazole; and mercaptobenzthiazole; isothiazole derivatives such as 5-chloro-2-methyl-4-isothiazoline-3-one and the magnesium salts thereof; 2-methyl-4-isothiazoline-3-one; 1,2-benzisothiazoline-3-one and the alkali metal, ammonium and amine salts thereof; and 2-n-octyl-4-isothiasoline-3-one; nitro compounds such as tris(hydroxymethyl)nitromethane; 5-bromo-5-nitro-1,3-dioxane and 2-bromo-2-nitropropane-1,3-diol; aldehydes and derivatives such as gluteraldehyde (pentanedial) p-chlorophenyl-3-iodopropargyl formaldehyde and glyoxal; amides such as chloracetamide; N,N-bis-(hydroxymethyl)chloracetamide; N-hydroxymethyl-chloracetamide and dithio-2,2-bis(benzmethyl amide); guanidine derivatives such as poly hexamethylene biguanide and 1,6-hexamethylene-bis[5-(4-chlorophenyl)-biguanide]; thiones such as 3,5-dimethyltetrahydro-1,3,5-2H-thiodiazine-2-thione; triazine derivatives such as hexahydrotriazine and 1,3,5-tri-(hydroxyethyl)-1,3,5-hexahydrotriazine; oxazolidine and derivatives thereof such as bis-oxazolidine; furan and derivatives thereof such as 2,5-dihydro-2,5-dialkoxy-2,5-dialkylfuran; carboxylic acids and the salts and esters thereof such as sorbic acid and the salts thereof and 4-hydroxybenzoic acid and the salts and esters thereof; phenol and derivatives thereof such as 5-chloro-2-(2,4-dichloro-phenoxy)phenol; thio-bis(4-chlorophenol) and 2-phenylphenol; sulphone derivatives such as diiodomethyl-paratolyl sulphone, 2, 3, 5, 6-tetrachloro-4 (methylsulphonyl) pyridine and hexachlorodimethyl sulphone.

The metal complex of the present invention is particularly useful when incorporated into a surface coating composition and hence, if used with other compounds having antimicrobial characteristics, these other compounds are advantageously compounds of the type used in surface coating compositions. Compounds which may be used in surface coating compositions include, inter-alia, anti-bacterial agents such as imidazolidinyl urea; 1, 2-dibromo-2, 4-dicyanobutane; 5-chloro-2-methyl-4-isothiazoline-3-one and the magnesium salts thereof; 2-methyl-4-isothiazolin-3-one; 1, 2-benzisothiazolin-3-one and the salts thereof, 2-bromo-2-nitropropane-1, 3-diol; gluteraldehyde; poly hexamethylene biguanide; triazine derivatives and oxazolidine and derivatives thereof. Surface coating compositions may also include anti-fungal agents such as 1 [2-(2, 4-dichlorophenyl)-2- (2-propenyloxy) ethyl] -1H-imidazole; 2-(methoxycarbonylamine) - benzimidazole; 2, 4, 5, 6 -tetrachloroisophthalodinitrile; zinc-2-pyridinethiol-N-oxide; 2-(thiocyanomethylthio)-benzthiazole; 2-n-octyl-4-thiazolin-3-one; dithio-2, 2-bis (benzmethyl amide); diiodomethyl - paratolylsulphone and 2, 3, 5, 6-tetrachloro-4 (methylsulphonyl) pyridine.

Further aspects of the present invention are described in the following illustrative examples. In the following tests and examples, all parts are by weight unless stated to the contrary.

In the following examples, the products obtained were subjected to microbiostatic evaluation and some products were also subjected to evaluation as preservatives against bacteria in paint and also, or alternatively, in metal working fluids. The microbiological testing was effected, under sterile conditions throughout, as follows:

In the microbiological testing, the products were tested for anti-microbial activity against bacteria and/or fungi. The bacteria used were one or more of Escherichia coli, Staphylococcus aureus, and Pseudomanas aeruginosa.The fungi used were one or more of Aspergillus niger, Aureobasidum pullulans, Cladosporium sphaerospermum, Aspergillus versicolor, and Chaetomium globosum.

These test organisms will be referred to hereafter as EC, SA, PA, AN, AP, CS, AV and CG respectively.

**Microbiostatic evaluation**

The material to be tested was dissolved in a suitable solvent and the solution obtained diluted with a further quantity of the same solvent to give a desired product concentration.

To a suitable agar medium was added a quantity of the product solution to give a desired concentration of the product. The agar medium containing the product was poured into petri dish plates and allowed to set.

The test organisms were surface inoculated onto the test plates by means of a multi-point inoculator. Each test plate was inoculated with both bacteria and fungi. The plates were incubated for four days at 25°C.

At the end of the incubation period, the plates were assessed visually for growth of the micro-organisms. The concentration of the product which inhibited the growth of a particular micro-organism was

recorded.

**Example 1**

A solution of 1.09 parts of zinc acetate in 50 parts of methanol was added, with stirring, at 20°C to a solution in 50 parts of methanol of 1.67 parts of N-methyl-N-hydroxythiobenzamide (prepared as described in Acta Chemica Scand 1967 (21) 1936). The mixture was stirred, without heating, for one hour. A white precipitate was formed which was separated by filtration. The separated precipitate was then washed with water at 15°C and subsequently with methanol. The washed precipitate was then dried in a vacuum oven at 15-20mm of mercury pressure and 25°C for 24 hours.

1.73 parts of precipitate were obtained which had the following analysis: C 47.9% wt; H 4.2% wt; N 7.0% wt and Zn 16.2% wt. the 2:1 zinc complex, $(C_8H_8NOS)_2Zn$, requires C 48.4% wt; H 4.0% wt; N 7.1% wt and Zn 16.4% wt.

**Comparative Example A**

The procedure of Example 1 was repeated with the exception that 0.99 parts of cupric acetate were used in place of 1.09 parts of zinc acetate to prepare the 2:1 copper complex. This complex had the analysis: C 47.7% wt; H 3.8% wt; N 6.8% wt; S 14.7% wt and Cu 15.7% wt. the 2:1 cupric complex, $(C_8H_8NOS)_2Cu$ requires C 48.6% wt; H 4.1% wt; N 7.1% wt; S 16.2% wt and Cu 15.9% wt.

**Comparative Example B**

0.5 parts of N-methyl-N-hydroxythiobenzamide were stirred in one part of ethanol. Four parts of a saturated aqueous solution of ferrous sulphate were then added dropwise over a period of ten minutes, the mixture was stirred for four hours at 20-25°C and was then allowed to stand for 16 hours at 20-25°C. A dark solid separated out and this was collected by filtration, washed with ten parts of water at 15°C, then with ethanol at 15°C and then dried at 15-20mm of mercury pressure and 25°C for 24 hours. The solid was collected by filtration and dried at 15-20mm of mercury pressure and 25°C for 24 hours.

0.39 parts of a dark crystalline solid having a melting point of 195-196°C were obtained.

The solid had the analysis:- C 51.5% wt, H 4.4% wt; N 7.2% wt and Fe 10.4% wt. The 3:1 ferric complex $(C_8H_8NOS)_3Fe$ requires C 52.0% wt; H 4.3% wt; N 7.6% wt; and Fe 10.1% wt.

Example 2

The complex of Example 1 and the complexes of Comparative Examples A and B were evaluated against a range of micro-organisms. In microbiostatic evaluation, as hereinbefore described, control of the test organisms was obtained at the levels set out in Table One.

TABLE ONE

| Test Organisms (a) | Complex (b) | | |
|---|---|---|---|
| | 1 | A | B |
| EC | 25 | 500 | 500 |
| SA | 500 | NA | 500 |
| PA | 500 | NA | NA |
| AN | 25 | NA | 500 |
| AP | 5 | 25 | 25 |
| CS | 5 | 500 | 25 |
| AV | 5 | 25 | 25 |
| CG | 5 | 25 | 25 |

**Notes to Table One**

(a) Organisms are as previously defined herein
(b) 1 is the complex of Example 1.
A is the complex of Comparative Example A.
B is the complex of Comparative Example B.
NA indicates the complex was not active at the highest level tested (500 ppm). The lowest level tested was 5 ppm.

Example 3

16.1 parts of N-methylhydroxylamine hydrochloride, 9 parts of anhydrous sodium acetate and 50 parts of acetic anhydride were mixed and heated under reflux for one minute. The mixture was allowed to cool to 50°C, filtered and the filtrate was drowned in water at 0-5°C. The aqueous solution obtained was stored for 16 hours at 20-25°C, neutralised by the addition of solid sodium carbonate and then extracted with chloroform. The chloroform extract was dried using anhydrous magnesium sulphate and then evaporated to dryness at 40°C and a pressure of 20mm of mercury in a rotary evaporator. An oil was obtained which was purified by flash chromatography by collecting the fraction eluted with a mixture of 15-50 volume of chloroform in a petroleum ether fraction having a boiling point in the range 40-60°C.

9.84 parts of N, O-diacetyl-N-methylhydroxylamine was obtained as a colourless oil having a nitrogen content of 11.0% wt (theoretical nitrogen content 10.7% wt).

4.4 parts of the foregoing, intermediate product and 8.3 parts of Lawesson's reagent (obtained from Fluka) were added to 50 parts of toluene and the mixture was stirred at 45-55°C for ten hours. The mixture was evaporated to dryness and the product was purified by flash chromatography, the product being the fraction eluted with a mixture of 22.5-32.5% volume of chloroform in a petroleum ether fraction having a boiling point range of 40-60°C.

5.48 parts of the product thus separated were added to 200 parts of water and the mixture was stirred at 50°C. Solid sodium carbonate was added to the mixture in a sufficient quantity to raise the pH to 9. The mixture at pH9 was stirred for one hour at 50°C, concentrated hydrochloric acid was then added dropwise to adjust the pH to pH of 7 and the neutral solution was then extracted with chloroform.

The chloroform extract was dried using anhydrous magnesium sulphate and was then evaporated to dryness. A syrup remained which was purified by flash chromatography, the fraction eluted with a mixture of 40-60% volume of chloroform in a petroleum ether fraction having boiling point range of 40-60°C.

1.92 parts of the eluted fraction (N-methyl-N-hydroxythioacetamide) were dissolved in 30 parts of methanol, the mixture was filtered and a solution of 2.8 parts of zinc acetate hydrate in 20 parts of water

were added. 2N aqueous sodium carbonate solution was added dropwise until the pH of the mixture was 7.5 and this mixture was then stirred for 16 hours at 20-25°C. The pH of the mixture was then adjusted to 5 by the dropwise addition of 2N hydrochloric acid. A solid was formed which was collected by filtration and dried at a pressure of 15-20mm of mercury and a temperature of 25°C for at least 24 hours.

The solid was then dissolved in 50 parts of boiling chloroform, the solution was treated with charcoal, filtered and 150 parts of petroleum ether of boiling point 40-60°C were then added. The mixture was allowed to stand for 16 hours at 20-25°C and a solid product was then collected by filtration, washed with a petroleum ether fraction of boiling point 40-60°C and then dried.

0.47 parts of a white solid having a melting point greater than 250°C was obtained.

The solid head the analysis C 25.9% wt; H 4.5% wt; N 10.0% wt; S 22.9% wt and Zn 22.5% wt. The 2:1 zinc complex $(C_3H_6NOS)_2Zn$ requires C 26.3% wt; H 4.4% wt, N 10.2% wt, S 23.4% wt and Zn 23.9% wt.

## Example 4

The procedure of Example 3 was repeated with the exception that the acetic anhydride was replaced by an equivalent amount of propionic anhydride. The 2;1 complex of N-hydroxy-N-methythiopropionamide with Zn was obtained as white crystals having a melting point of 126-128°C.

The solid had the analysis C 32.1% wt; H 5.6% wt; N 8.9% wt; S 21.5% wt and Zn 20.2% wt. The zinc complex $(C_4H_8NOS)_2$ Zn requires C 31.9% wt; H 5.3% wt; N 9.3% wt; S 21.3% wt and Zn 21.6% wt.

## Example 5

The procedure of Example 3 was repeated with the exception that the N-methylhydroxylamine was replaced by an equivalent amount of N-benzylhydroxylamine. The 2:1 complex of N-benzyl-N-hydroxythioacetamide with Zn was obtained as white crystals of melting point 189-192°C.

The solid had the analysis C 51.0% wt; H 4.9% wt; N 6.4% wt; S 14.8% wt and Zn 14.9% wt. The zinc complex $(C_9H_{10}NOS)_2$ Zn requires C 50.8% wt; H 4.7% wt; N 6.6 % wt, S 15.1% wt and Zn 15.3 wt.

## Example 6

The procedure of Example 3 was repeated with the exception that the acetic anhydride was replaced by an equivalent amount of isobutyric anhydride. The 2:1 complex of N-methyl-N-hydroxythioisobutyramide with Zn was obtained as white crystals having a melting point of 168-168.5°C.

The solid had the analysis c 36.4% wt; H 6.2% wt; N 8.2% wt; S 19.2% wt and Zn 19.6% wt. The zinc complex $(C_5H_{10}NOS)_2$ Zn requires C 36.4% wt; H 6.1% wt N 8.5% wt; S 19.4% wt and Zn 19.9% wt.

## Example 7

The procedure of Example 3 was repeated with the exception that the acetic anhydride was replaced by an equivalent amount of butyric anhydride. The 2:1 complex of N-methyl-N-hydroxythiobutyramide with zinc was obtained as a solid, which could not be obtained in a crystalline form.

The solid had the analysis C 36.8% wt and H 6.4% wt. The zinc complex $(C_5H_{10}NOS)_2$ Zn requires C 36.4% wt and H 6.1% wt.

## Examples 8 to 12

The complexes of Examples 3 to 7 were evaluated against a range of micro-organisms using the microbiostatic evaluation procedure hereinbefore described. Control of the test organisms was obtained at the levels set out in Table Two.

TABLE TWO

| TEST ORGANISM (a) | COMPLEX (b) (c) (d) | | | | |
|---|---|---|---|---|---|
| | 3 | 4 | 5 | 6 | 7 |
| EC | 10 | 10 | 500 | 25 | 10 |
| SA | 2 | 10 | 500 | 10 | 10 |
| PA | 250 | 250 | NA | 250 | 250 |
| AN | 25* | 25 | 500 | 25* | 25* |
| AP | 500 | 5 | 500 | 25* | 25* |
| CS | 25* | 25 | 500 | 25* | 25* |
| AV | 25* | 5 | 500 | 25* | 25* |
| CG | 25* | 25 | 500 | 20* | 25* |

Notes to Table Two

(a) and (b) are both as defined in Notes to Table One.

(c) 3, 4, 5, 6 and 7 are the complexes of Examples 3, 4, 5, 6 and 7 respectively.
(d) * the lowest level tested was 25 ppm.

**Example 13**

The procedure of example 1 was repeated with the exception that 1.6 parts of silver nitrate were used in place of 1.09 parts of zinc acetate to prepare the 1:1 silver complex of N-methyl-N-hydroxythiobenzamide as an amorphous powder. This complex was found to contain 41.4% wt of silver. The 1:1 silver complex $(C_8H_8NOS)Ag$ requires 39.4% weight of silver.

**Example 14**

The silver complex of Example 13 was evaluated against a range of micro-organisms using the microbiostatic evaluation procedure hereinbefore described. Control of the test organisms was obtained at the levels set out in Table Three.

TABLE THREE

| TEST ORGANISM (a) (e) | COMPLEX AMOUNT (f) |
|---|---|
| AN | 100 |
| CA | 25 |
| AP | 100 |
| GR | 100 |
| PP | 100 |
| EC | 25 |
| PA | 25 |
| SA | 25 |
| BS | 25 |

Notes to Table Three

(a) AN, AP, EC, PA and SA are as defined in Notes to Table One.

(e) CA is candida alibicans ( a yeast)
GR is Gliocladium roseum (a fungus)
PP is Penicillium pinophilum (a fungus)
BS is Bacillus subtiles (a bacterium)

(f) The complex was tested at levels of 100 ppm and 25 ppm only.

**Examples 15 to 19**

An acrylic latex (Revacryl 1A latex) was mixed with sterile distilled water in the proportions, by volume of 3:1. 50 g aliquots of the latex/water mixture containing 0.2% yeast extract and the complexes of Examples 3 to 7 were mixed and stored in sealed containers for one week at 40°C. The stored mixtures were allowed to cool to ambient temperature.

An inoculum of a mixed suspension of bacteria was prepared by mixing equal amounts of suspensions each of which contained a different one of the bacteria Aeromonas hydrophila, Proteus rettgeri, Pseudomonas aeruginosa, Serratia marcescens, Alcaligenes spp, Pseudomanes cepacia, Pseudomonas putida.

Each latex mixture was inoculated with 1cm$^3$ of the mixed bacterial suspension and incubated at 30°C. After contact times of one, three and seven days, a small aliquot of the latex mixture was removed and examined for bacterial growth. The lowest level of complex at which no growth of bacteria was detected was recorded. After removal of the seven day aliquot, a further 1cm$^3$ of the mixed bacterial suspension was added. Aliquots were removed after one, three and seven days of the second week. At the end of the second week, a further 1cm$^3$ of the mixed bacterial suspension was added. Aliquots were removed after one, three and seven days of the third week. The results obtained are set out hereafter in Table Four.

TABLE FOUR

| EX | COMPLEX (c) | WEEK 1 | | | WEEK 2 | | | WEEK 3 | | |
|---|---|---|---|---|---|---|---|---|---|---|
| | | DAY (h) | | | DAY (h) | | | DAY (h) | | |
| | | 1 | 3 | 7 | 1 | 3 | 7 | 1 | 3 | 7 |
| 15 | 3 | GT500 | 500 | LT50 | 500 | 500 | 250 | 500 | 250 | 250 |
| 16 | 4 | GT500 | GT500 | 250 | GT500 | 500 | 250 | GT500 | GT500 | 250 |
| 17 | 5 | GT500 | GT500 | GT500 | GT500 | GT500 | GT500 | GT500 | GT500 | GT500 |
| 18 | 6 | GT500 | 500 | LT50 | GT500 | 500 | 250 | GT500 | 500 | 250 |
| 19 | 7 | 500 | 500 | LT50 | GT500 | 500 | LT50 | GT500 | GT500 | LT50 |

**Notes to Table Four**

(c) is as defined in Notes to Table Two.

(h) The complexes were present in amounts of 50, 250 and 500 ppm. GT indicates growth occurred at the maximum level tested. LT indicates no growth occurred at the minimum level tested.

**Examples 20 to 24**

The complexes of Examples 3 to 7 were mixed with 50g aliquots of an exterior acrylic emulsion paint (based on Revacryl 1A latex at pH9) containing 0.2% yeast extract and the mixtures obtained were stored in sealed containers for one week at 40°C. The stored mixtures were allowed to cool to ambient temperature.

Each paint mixture was inoculated with 1cm$^3$ of the mixed bacterial suspension as described in Examples 15 to 19 and incubated at 30°C. The mixtures were examined for bacterial growth and further inoculated as described in Examples 15 to 19. The results obtained are set out hereafter in Table Five.

TABLE FIVE

| EX | COMPLEX (c) | WEEK 1 | | | WEEK 2 | | | WEEK 3 | | |
|---|---|---|---|---|---|---|---|---|---|---|
| | | DAY (h) | | | DAY (h) | | | DAY (h) | | |
| | | 1 | 3 | 7 | 1 | 3 | 7 | 1 | 3 | 7 |
| 20 | 3 | GT500 | 250 | 250 | GT500 | 250 | 250 | GT500 | 500 | 500 |
| 21 | 4 | GT500 | 250 | 250 | GT500 | 500 | 500 | GT500 | 500 | 500 |
| 22 | 5 | GT500 | GT500 | GT500 | GT500 | GT500 | GT500 | GT500 | GT500 | GT500 |
| 23 | 6 | GT500 | 250 | 250 | GT500 | 250 | 250 | GT500 | 250 | 250 |
| 24 | 7 | GT500 | 50 | LT50 | GT500 | 250 | LT50 | GT500 | 250 | LT50 |

**Notes to Table Five**

(c) is as defined in Notes to Table Two

(h) is as defined in Notes to Table Four

**Example 25**

To samples of a metal working fluid (Burmah Castrol Cooledge B.I) were added portions of the product of Example 1 to give concentrations in the metal working fluid of 0.125%; 0.25% and 0.5% w/v of the product of Example 1. After storing the mixtures at 50°C for seven days, each mixture was diluted with

deionised water in the proportion, by volume, of 1:19 mixture to water.

To $100cm^3$ of the mixture obtained as described was added, at intervals, an aliquot ($0.5cm^3$) of a cell suspension from a 24 hour broth of Pseudomonas aeruginosa. The mixture was incubated at 30°C and samples of the mixture were taken for a cell count at intervals. The results obtained are set out in Table Six.

TABLE SIX

| COMPLEX CONC$^n$ (i) | CELL COUNT (j) (k) | | |
|---|---|---|---|
| | 6 days | 14 days | 21 days |
| 0.5 | NIL | NIL | $1.2 \times 10^2$ |
| 0.25 | NIL | NIL | NIL |
| 0.125 | NIL | NIL | NIL |

**Notes to Table Six**

(i) Complex conc$^n$ is the % w/v of the complex relative to the metal working fluid.

(j) The cell count was determined using the procedure described by A.L.Koch on pages 185-187 of "Manual of Methods for General Bacteriology" published by the American Society for Microbiology.

(k) The inoculum for the first week was obtained by diluting the broth by a factor of $10^4$, the inoculum for the second week was obtained by diluting the broth by a factor of $10^2$ and for the third week the inoculum was he undiluted broth.

**Example 26**

The procedure of Example 25 was repeated with the exception that a different metal working fluid (Shell Dromus B Oil) was used with the product of Example 3. The results obtained are set out in Table Seven.

TABLE SEVEN

| COMPLEX CONC$^n$ (i) | CELL COUNT (j) (l) | | | | |
|---|---|---|---|---|---|
| | 7 days | 14 days | 21 days | 28 days | 35 days |
| 0.5 | NIL | NIL | NIL | NIL | NIL |
| 0.25 | NIL | NIL | NIL | NIL | $3.7 \times 10^3$ |
| 0.125 | NIL | NIL | NIL | $3.1 \times 10^6$ | $5.1 \times 10^6$ |

**Notes to Table Seven**

(i) and (j) are as defined in Notes to Table Six.

(l) The inoculum for the first week was obtained by diluting the broth by a factor of $10^3$, for the second week the broth was diluted by a factor of $10^2$ and for the third, fourth and fifth weeks the inoculum was the undiluted brot .

**Claims**

1. A complex of the formula:
$[R^1CSNRO]_xM(L)_y$
where
R and $R^1$ may be the same or different and are hydrocarbyl groups, or substituted hydrocarbyl groups;
M is a metal of Group IIIA to Va or IB to VIIB, with the exception of copper;
L represents a ligand;
x has a value of from 1 to 3; and
y is zero or has a value of from 1 to 4.

2. A complex as claimed in claim 1 wherein R is a lower alkyl group and $R^1$ is a lower alkyl group or a phenyl group.

3. A complex as claimed in either claim 1 or claim 2 wherein M is zinc or silver.

4. A complex as claimed in any one of claims 1 to 3 wherein x corresponds to the valency of the metal M.

5. A 2:1 complex of
N-methyl-N-hydroxythiobenzamide and zinc;
N-methyl-N-hydroxythioacetamide and zinc;
N-methyl-N-hydroxythiopropionamide and zinc;
N-methyl-N-hydroxythioisobutyramide and zinc or
N-methyl-N-hydroxythiobutyramide and zinc,
or a 1:1 complex of
N-methyl-N-hydroxythiobenzamide and silver.

6. A process for the preparation of a complex of the formula
$[R^1CSNRO]_xM(L)_y$
which comprises reacting a solution of a salt of M with an N-hydroxythioamide of the formula
$R^1CSNROH$
wherein
R and $R^1$ may be the same or different and are hydrocarbyl groups, or substituted hydrocarbyl groups;
M is a metal of Group IIA to VA or IB to VIIB, with the exception of copper;
L represents a ligand;
x has a value of from 1 to 3; and
y is zero or has a value of from 1 to 4.

7. A process as claimed in claim 6 wherein an aqueous or methanolic solution of zinc acetate is mixed with a methanolic solution of N-methyl-N-hydroxythiobenzamide, N-methyl-N-hydroxythioacetamide; N-methyl-N-hydroxythipropionamide; N-methyl-N-hydroxythioisobutyramide or N-methyl-N-hydroxythiobutyramide.

8. A biocide composition comprising a carrier and an effective amount of at least one complex as claimed in any one of claims 1 to 5.

9. A composition as claimed in claim 8 which contains from 0.0001% up to 30% by weight of the at least one complex.

10. A method for inhibiting the growth of micro-organisms on, or in, a medium which comprises treating the medium with a complex as claimed in any one of claims 1 to 5 or a composition as claimed in either claim 8 or claim 9.

11. A surface coating composition or a metal working fluid which contains at least one complex as claimed in any one of claims 1 to 5.

Claims for the following Contracting States : ES

1. A biocide composition comprising a carrier and an effective amount of at least one complex of the formula:
$[R^1CSNRO]_xM(L)_y$
where
R and $R^1$ may be the same or different and are hydrocarbyl groups, or substituted hydrocarbyl groups;
M is a metal of Group IIIA to Va or IB to VIIB, with the exception of copper;
L represents a ligand;
x has a value of from 1 to 3; and
y is zero or has a value of from 1 to 4.

2. A composition as claimed in claim 1 which is a suspension or emulsion of the at least one complex

and a liquid carrier.

3. A composition as claimed in either claim 1 or 2 which contains from 0.0001% up to 30% by weight of the at least one complex.

4. A compisition as claimed in any one of claims 1 to 3 wherein the at least one complex is one in which R is a lower alkyl group and $R^1$ is a lower alkyl group or a phenyl group.

5. A composition as claimed in any one of claims 1 to 4 wherein the at least one complex is one in which M is zinc or silver.

6. A composition as claimed in any one of claims 1 to 5 wherein the at least one complex is one in which x corresponds to the valency of the metal M.

7. A biocide composition comprising a carrier and an effective amound of at least one complex which is a 2:1 complex of
N-methyl-N-hydroxythiobenzamide and zinc;
N-methyl-N-hydroxythioacetamide and zinc;
N-methyl-N-hydroxythiopropionamide and zinc;
N-methyl-N-hydroxythioisobutyramide and zinc or
N-methyl-N-hydroxythiobutyramide and zinc,
or a 1:1 complex of
N-methyl-N-hydroxythiobenzamide and silver.

8. A process for the preparation of a complex of the formula
$[R^1CSNRO]_xM(L)_y$
which comprises reacting a solution of a salt of M with an N-hydroxythioamide of the formula
$R^1CSNROH$
wherein
R and $R^1$ may be the same or different and are hydrocarbyl groups, or substituted hydrocarbyl groups;
M is a metal of Group IIA to VA or IB to VIIB, with the exception of copper;
L represents a ligand;
x has a value of from 1 to 3; and
y is zero or has a value of from 1 to 4.

9. A process as claimed in claim 8 wherein an aqueous or methanolic solution of zinc acetate is mixed with a methanolic solution of N-methyl-N-hydroxythiobenzamide, N-methyl-N-hydroxythioacetamide; N-methyl-N-hydroxythipropionamide; N-methyl-N-hydroxythioisobutyramide or N-methyl-N-hydroxythiobutyramide.

10. A method for inhibiting the growth of micro-organisms on, or in, a medium which comprises treating the medium with a complex of the formula (I), or with a composition which contains an effective amount of at least one complex of the formula (I): $[R^1CSNRO]_xM(L)_y$
where
R and $R^1$ may be the same or different and are hydrocarbyl groups, or substituted hydrocarbyl groups;
M is a metal of Group IIIA to Va or IB to VIIB, with the exception of copper;
L represents a ligand;
x has a value of from 1 to 3; and
y is zero or has a value of from 1 to 4.

11. A surface coating composition or a metal working fluid which contains at least one complex of the formula:
$[R^1CSNRO]_xM(L)_y$
where
R and $R^1$ may be the same or different and are hydrocarbyl groups, or substituted hydrocarbyl groups;
M is a metal of Group IIIA to Va or IB to VIIB, with the exception of copper;
L represents a ligand;
x has a value of from 1 to 3; and
y is zero or has a value of from 1 to 4.

European Patent Office

# EUROPEAN SEARCH REPORT

Application number

| DOCUMENTS CONSIDERED TO BE RELEVANT | | | EP 90300430.7 |
|---|---|---|---|
| Category | Citation of document with indication, where appropriate, of relevant passages | Relevant to claim | CLASSIFICATION OF THE APPLICATION (Int Cl 5) |
| A | METHODEN DER ORGANISCHEN CHEMIE, vol. E5, part II, 1985 Georg Thieme Verlag, Stuttgart W. BAUER, K. KÜHLEIN "Thio-carbonsäure-hydroxamide, -S-ester-hydroximide" pages 1278-1287 * Page 1286, lines 10-16; page 1287, lines 1-6 * -- | 1 | C 07 C 327/38<br>A 01 N 55/02<br>A 01 N 37/28 |
| D,A | EP - A2 - 0 249 328 (IMPERIAL CHEMICAL INDUSTRIES) * Claims 1,4-7 * -- | 1,8, 10,11 | |
| A | CHEMICAL ABSTRACTS, vol. 107, no. 9, August 31, 1987, Columbus, Ohio, USA S.P. MATHUR et al. "Preparation and insecticidal activity of thioanalogs of hydroxylamine derivatives" page 222, abstract-no. 72 422q & Acta Cienc. Indica, Chem. 1985, 11(2), 124-5 -- | 1 | TECHNICAL FIELDS SEARCHED (Int Cl 5)<br>A 01 N<br>C 07 C 327/00 |
| D,A | ACTA CHEMICA SCANDINAVICA, vol. 21, part II, 1967 K.A. JENSEN et al. "Studies of Thioacids and Their Derivatives. XIII. Thiohydroxamic Acids" pages 1936-1941 * Abstract * -- | 1 | |
| A | CHEMICAL ABSTRACTS, vol. 93, no. 18, November 3, 1980, Columbus, Ohio, USA D.J. BROCKWAY et al. "An electrochemical study of thio- | 1,8 | |

The present search report has been drawn up for all claims

| Place of search | Date of completion of the search | Examiner |
|---|---|---|
| VIENNA | 27-03-1990 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number

-2-

EP 90300430.7

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| | hydroxamate and hydroxamate complexes of iron (III)" page 514, abstract-no. 175 976v & J.Chem.Soc., Dalton Trans. 1980, (7), 1112-17 -- | | |
| A | CHEMICAL ABSTRACTS, vol. 106, no. 15, April 13, 1987, Columbus, Ohio, USA R.M. COATES et al. "Thioimidate N-oxides: nitrones of thioesters" page 587, abstract-no. 119 280p & J.Org.Chem. 1987, 51(26), 5198-209 -- | 1,8 | |
| A | CHEMICAL ABSTRACTS, vol. 102, no. 16, April 22, 1985, Columbus, Ohio, USA FUJI PHOTO FILM CO., LTD "Photographic releasing agents for blocked photographic reagents" page 582, abstract-no. 140 714k & Jpn.Kokai Tokkyo Koho JP 59,198,453 (84,198,453) ---- | 1,8 | TECHNICAL FIELDS SEARCHED (Int Cl 5) |

The present search report has been drawn up for all claims

| Place of search | Date of completion of the search | Examiner |
|---|---|---|
| VIENNA | 27-03-1990 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document